(19) Europäisches Patentamt
European Patent Office
Office européen des brevets

(11) Veröffentlichungsnummer: **0 002 246**
**B1**

(12) # EUROPÄISCHE PATENTSCHRIFT

(45) Veröffentlichungstag der Patentschrift: **20.01.82**

(21) Anmeldenummer: **78101467.5**

(22) Anmeldetag: **28.11.78**

(51) Int. Cl.³: **C 07 C 131/00,**
A 01 N 35/10, A 01 N 39/02

(54) **Oximester, diese enthaltende Unkrautbekämpfungsmittel, deren Verwendung als Unkrautbekämpfungsmittel sowie ein Verfahren zu deren Herstellung.**

(30) Priorität: **28.11.77 LU 78591**
**15.09.78 CH 9667/78**

(43) Veröffentlichungstag der Anmeldung:
**13.06.79 Patentblatt 79/12**

(45) Bekanntmachung des Hinweises auf die Patenterteilung:
**20.01.82 Patentblatt 82/3**

(84) Benannte Vertragsstaaten:
**BE CH DE FR GB LU NL SE**

(56) Entgegenhaltungen:
**NL - A - 78 01120**
**US - A - 3 925 473**

**R. Wegler "Chemie der Planzenschutz- und Schädlingsbekämpfungsmittel", Band 5, 1977, Seiten 190—191**

(73) Patentinhaber: **F. HOFFMANN-LA ROCHE & CO. Aktiengesellschaft**
**CH-4002 Basel (CH)**

(72) Erfinder: **Milos, Suchy, Dr.**
**Grossplatzstrasse 3**
**CH-8122 (CH)**

(74) Vertreter: **Lederer, Franz, Dr. et al,**
**Patentanwälte Lederer, Meyer Lucile-Grahn-Strasse 22**
**D-8000 München 80 (DE)**

**EP 0 002 246 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Oximester, diese enthaltende Unkrautbekämpfungsmittel, deren Verwendung als Unkrautbekämpfungsmittel sowie ein Verfahren zu deren Herstellung

Die Erfindung betrifft Verbindungen der allgemeinen Formel

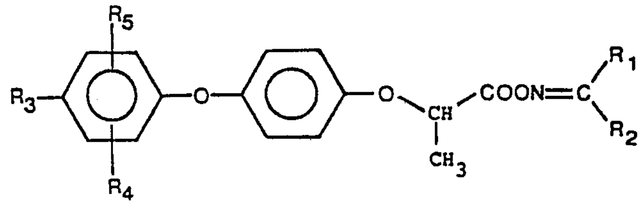

I

worin $R_1$ Wasserstoff, Alkyl mit 1—6 Kohlenstoffatomen oder Phenyl, $R_2$ Wasserstoff, Alkyl mit 1—6 Kohlenstoffatomen, Alkenyl mit 2—6 Kohlenstoffatomen, Alkinyl mit 2—6 Kohlenstoffatomen oder Phenyl oder $R_1$ und $R_2$ zusammen mit dem Kohlenstoffatom, an das sie geknüpft sind, einen Cyclohexanring bilden, der gegebenenfalls mono-, di- oder tri-Alkyl mit 1—3 Kohlenstoffatomen substituiert sein kann, $R_3$ Wasserstoff, Fluor, Chlor, Brom, Trifluormethyl oder Nitro und $R_4$ und $R_5$ Wasserstoff oder Chlor darstellen, mit der Massgabe, das $R_1$ und $R_2$ nicht gleichzeitig Wasserstoff bedeuten.

Die Erfindung betrifft ferner ein Verfahren zur Herstellung von Verbindungen der Formel I, Verbindungen der Formel I als Unkrautbekämpfungsmittel, Unkrautbekämpfungsmittel, die mindestens eine Verbindung der Formel I enthalten sowie die Verwendung solcher Verbindungen als Unkrautbekämpfungsmittel.

Auch die US-Patentschrift Nr. 3.925.473 offenbart Oximester von 2-Phenoxypropionsäuren, die sich als Unkrautbekämpfungsmittel eignen. Die erfindungsgemässen Verbindungen unterscheiden sich von den bekannten im wesentlichen dadurch, dass sie zwei Phenoxygruppen aufweisen. Ferner offenbar "Chemie der Pflanzenschutz- und Schädlingsbekämpfungsmittel", Band 5, Seite 191 (1977), substituierte 2-(p-Phenoxyphenoxy)propionsäuren und gewisse Alkylester davon, die sich ebenfalls als Unkrautbekämpfungsmittel eignen, aber im Gegensatz zu den neuen Oximestern I Alkancarbonsäuren und deren Ester darstellen.

Das Verfahren zur Herstellung von Verbindungen der Formel I ist dadurch gekennzeichnet, dass man

a) ein Säurechlorid oder ein reaktionsfähiges Anhydrid dieser Säure der Formel

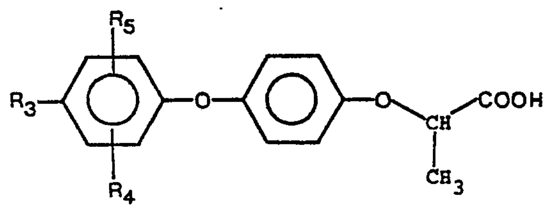

II

worin $R_3$, $R_4$ und $R_5$ die in Formel I angegebene Bedeutung besitzen, mit einem Oxim

$$R_1R_2CNOH \qquad III$$

worin $R_1$ und $R_2$ die in Formel I angegebene Bedeutung besitzen, umsetzt, oder

b) eine Verbindung der allgemeinen Formel

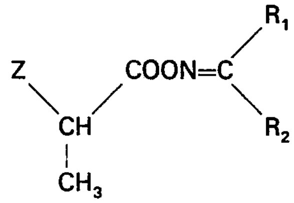

IV

worin Z Chlor, Brom, Jod, Mesyloxy oder Tosyloxy bedeutet und $R_1$ und $R_2$ die in Formel angegebene Bedeutung besitzen,
mit einer Verbindung der allgemeinen Formel

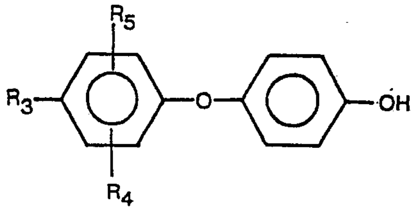

V

worin $R_3$, $R_4$ und $R_5$ die in Formel I angegebene Bedeutung besitzen,
oder einem Alkalimetallsalz davon, notwendigenfalls in Gegenwart einer Base, umsetzt, oder

c) eine Säure der Formel II mit einem Oxim der Formel III in Gegenwart von Dicyclohexylcarbodiimid umsetzt.

Der Ausdruck "Alkyl" mit 1—6 bzw. 1—3 Kohlenstoffatomen umfasst—wenn nicht anderweitig angegeben — sowohl geradkettige als auch verzweigte Kohlenwasserstoffradikale mit 1—6 bzw. 1—3 Kohlenstoffatomen, wie Methyl, Aethyl, Propyl, Isopropyl, Butyl, Isobutyl, tert.-Butyl und dgl.

Die Ausdrücke "Alkenyl" und "Alkinyl" mit 2—6 Kohlenstoffatomen umfassen ungesättigte geradkettige und verzweigte Kohlenwasserstoffradikale mit bis zu 6 Kohlenstoffatomen wie Allyl, Butenyl, Isobutenyl, Pentenyl, Isopentenyl und dgl. sowie Propargyl, Butinyl, Isobutinyl, Pentinyl und dgl.

Bevorzugte Verbindungen der Formel I sind solche, worin $R_1$ und $R_2$ Alkylreste mit 1—2 Kohlenstoffatomen oder einen Phenylrest und $R_3$ Chlor oder den Trifluormethylrest und $R_4$ und $R_5$ Wasserstoff bedeuten.

Besonders bevorzugte Verbindungen der Formel I sind:

Aceton-O-/2-[p-(p-Chlorphenoxy)phenoxy]propionyl/-oxim,
2-Butanon-O-/2-[p-(p-Chlorphenoxy)phenoxy]propionyl/-oxim,
Benzaldehyd-O-/2-[p-(p-Chlorphenoxy)phenoxy]propionyl/-oxim,
2-(6-Methyl-5-heptenon)-O-/2-[p-(p-chlorphenoxy)phenoxy]propionyl/-oxim,
6-Undecanon-O-/2-[p-(p-chlorphenoxy)phenoxy]propionyl/-oxim,
Cyclohexanon-O-/2-[p-(p-chlorphenoxy)phenoxy]propionyl/-oxim,
Aceton-O-D-/2-[p-(p-chlorphenoxy)phenoxy]propionyl/-oxim,
2-(3-Butynon)-O-/2-[p-(p-chlorphenoxy)phenoxy]propionyl/-oxim,
Aceton-O-/2-[p-(p-trifluormethylphenoxy)phenoxy]-propionyl/-oxim,
Acetophenon-O-/2-[p-(p-trifluormethylphenoxy)phenoxy]-propionyl/-oxim,
2-Butanon-O-/2-[p-(p-trifluormethylphenoxy)phenoxy]-propionyl/-oxim,
6-Undecanon-O-/2-[p-(p-trifluormethylphenoxy)phenoxy]-propionyl/-oxim,
Benzaldehyd-O-/2-[p-(p-trifluormethylphenoxy)phenoxy]-propionyl/-oxim,
Aceton-O-D-/2-[p-(p-trifluormethylphenoxy)phenoxy]-propionyl/-oxim,
Aceton-O-/2-[p-(2,4-dichlorphenoxy)phenoxy]propionyl/-oxim,
Aceton-O-/2-[p-(p-Bromphenoxy)phenoxy]propionyl/-oxim,
Aceton-O-/2-[p-(3,4-dichlorphenoxy)phenoxy]propionyl/-oxim,
Aceton-O-/2-[p-(2,6-dichlorphenoxy)phenoxy]propionyl/-oxim.

In einer Ausführungsform zur Herstellung von substituierten Phenoxyalkancarbonsäureoximestern der allgemeinen Formel I wird ein reaktionsfähiges Derivat einer Säure der Formel II mit einem Oxim der Formel III umgesetzt.

Der Ausdruck "reaktionsfähiges Derivat der Säure" bedeutet ein Säurehalogenid oder ein Säureanhydrid.

Zur Herstellung der substituierten Phenoxyalkancarbonsäureoximester der allgemeinen Formel I wird die Veresterung der Säure der allgemeinen Formel II mit dem Oxim der allgemeinen Formel III vorzugsweise in einem geeigneten inerten Lösungsmittel, besonders bevorzugt bei Raumtemperatur oder erhöhter Temperatur, durchgeführt. Ein bevorzugter Temperaturbereich ist —10 bis +100°C, besonders bevorzugt ist +20 bis +70°C. Bei Verwendung eines Säurehalogenids als reaktionsfähiges Derivat der Säure wird die Umsetzung mit dem Oxim bei Raumtemperatur und in Gegenwart eines Säureacceptors, z.B. eines tertiären Amins, wie Pyridin oder Triäthylamin oder in alkalischer Lösung nach Schotten-Baumann durchgeführt. Man erhält in hoher Ausbeute den entsprechenden Ester. Als Säurehalogenide werden die Säurechloride bevorzugt. Die Umsetzung wird vorzugsweise in Gegenwart eines inerten Lösungsmittels durchgeführt, wie Benzol, Toluol, Petroläther oder nach Schotten-Baumann in alkalischer Lösung. Bei Verwendung des Anhydrides der Säure der allgemeinen Formel II kann der entsprechende Alkancarbonsäureester der allgemeinen Formel I in hoher Ausbeute durch Erhitzen des Anhydrides mit dem Oxim der allgemeinen Formel III in Gegenwart einer Base, vorzugsweise einem Alkalimetallcarbonat hergestellt werden. Besonders bevorzugt ist Natriumcarbonat.

In einer weiteren Ausführungsform zur Herstellung von Verbindungen der Formel I wird eine Ver-

bindung der Formel IV mit einem Alkalimetallsalz einer Verbindung der Formel V in an sich bekannter Weise umgesetzt. Die Umsetzung erfolgt zweckmässigerweise in einem inerten organischen Lösungsmittel wie Kohlenwasserstoffen, z.B. Benzol oder Toluol, Aethern, z.B. Diäthyläther, Tetrahydrofuran, Dimethoxyäthan oder Hexamethylphosphorsäuretriamid, und dgl. Temperatur und Druck sind nicht kritisch und man arbeitet bevorzugt bei einer Temperatur zwischen −20° und der Rückflusstemperatur der Reaktionsmischung, vorzugsweise zwischen −10° und 30°C.

In einer weiteren Ausführungsform zur Herstellung von Verbindungen der Formel I wird eine Säure der Formel II mit einem Oxim der Formel III in Gegenwart von Dicyclohexylcarbodiimid umgesetzt. Zu diesem Zweck wird die Säure der Formel II in einem inerten organischen Lösungsmittel wie chlorierte Kohlenwasserstoffe, beispielsweise Dichlormethan, Chloroform, Tetrachlorkohlenstoff oder Trichloräthan, Aether wie beispielsweise Diäthyläther, Diisopropyläther oder Dioxan, aromatische Kohlenwasserstoffe, beispielsweise Benzol, Toluol oder Xylol usw. gelöst und danach wird das Oxim der Formel III in diesem Gemisch suspendiert. Das Dicyclohexylcarbodiimid wird ebenefalls im bereits verwendeten Lösungsmittel gelöst und zum Reaktionsgemisch zugegeben. Die Umsetzung erfolgt in einem Temperaturbereich zwischen 0° und der Siedetemperatur des Reaktionsgemisches, vorzugsweise zwischen Raumtemperatur und 50°C. Nach ca. 2 Stunden ist die Umsetzung beendet, das Reaktionsgemisch wird filtriert und danach eingedampft. Der Rückstand wird gegebenenfalls zur Reinigung umkristallisiert oder chromatographiert.

Die substituierten Phenoxyalkancarbonsäureoximester der allgemeinen Formel I schliessen auch optische Isomere ein, da sie ein asymmetrisches Kohlenstoffatom in der $\alpha$-Stellung besitzen. Weitere asymmetrische Kohlenstoffatome können die Esterkomponenten enthalten. Erwünschtenfalls können die racemischen Verbindungen unter Verwendung bekannter Verfahren in rechtsdrehende und linksdrehende Verbindungen getrennt werden. Derartige Verfahren sind beispielsweise in Industrial and Engineering Chemistry *60* (8) 12—28 beschrieben. Die Isomeren und die racemischen Mischungen besitzen alle herbicide Aktivität, jedoch ist das Ausmass dieser Aktivität unterschiedlich. Am grössten ist die Aktivität beim D-Isomeren, danach folgt die racemische Mischung und dann das L-Isomere. Es wurde im Zusammenhang mit derartigen Untersuchungen gefunden, dass beispielsweise in bestimmten Versuchsanordnungen das D-Isomere des Aceton-O-/2-[p-(p-chlorphenoxy)phenoxy]-propionyl/oxim eine grössere Aktivität als die racemische Mischung aufweist.

Die Isomeren — insbesondere die D-Isomeren — können auch durch Synthese aus entsprechenden optisch aktiven Ausgangsmaterialien hergestellt werden.

Infolge der Stickstoff-Kohlenstoff-Doppelbindung in der

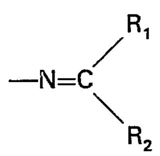

Gruppe erhält man jeweils zwei geometrische Isomere (wenn $R_1$ und $R_2$ unterschiedliche Bedeutung haben), die als syn- und anti-Form bezeichnet werden. Es gelingt in gewissen Fällen, derartige Isomere zu isolieren. Die Formel I soll demnach auch diese Isomeren umfassen.

Die vorliegende Erfindung betrifft auch herbicide Zusammensetzungen, die als aktiven Bestandteil ein oder mehrere Verbindungen der Formel I, wie oben definiert, enthalten. Die herbicide Zusammensetzung enthält zweckmässigerweise zumindest eines der folgenden Materialien: Trägerstoffe, Netzmittel, inerte Verdünnungsmittel und Lösungsmittel.

Die erfindungsgemässen Herbicide eignen sich besonders zur Bekämpfung von Ungräsern, insbesondere von Ackerfuchsschwanz (Alopecurus myosuroides) und Hirsearten wie beispielsweise Hühnerhirse (Echinochloa grus-galli), grosse Borstenhirse (Setaria faberii) und haarartige Hirse (Panicum capillare) in Getriede — insbesonders Gerste-, Hafer- und Weizen- sowie Reis-, Baumwolle-, Soya-Zuckerrüben und Gemüsekulturen. Besonders bevorzugt eignen sich die erfindungsgemässen Herbicide zur Bekämpfung von Ungräsern in Zuckerrübenkulturen. So besitzt beispielsweise das Aceton-O-/2-[p-(p-chlorphenoxy)phenoxy]propionyl/-oxim, das 2-Butanon-O-/2-[p-(p-chlorphenoxy)phenoxy]-propionyl/-oxim oder das Aceton-O-/2-[p-(p-trifluormethylphenoxy)phenoxy]propionyl/-oxim bei einer Konzentration von 1,25 kg/ha genügend Wirksamkeit gegen Ungräser, ohne jedoch die Zuckerrübenkulturen zu schädigen. Im allgemeinen genügt eine Konzentration von 0,1—6 kg/ha, vorzugsweise 0,6—2,0 kg/ha, besonders bevorzugt 1—1,5 kg/ha, um den gewünschten herbiciden Effekt mit Verbindungen der Formel I zu erzielen.

Für den Fall, dass $R_3$ den Trifluormethylrest darstellt, können diese Verbindungen nur bedingt im Getreidebau Verwendung finden, da etwas Phytotoxizität eintritt. Diese Verbindungen eignen sich jedoch besonders zur Bekämpfung von Ungräsern in Reis-, Baumwoll-, Soya-, Zuckerrüben- und Gemüsekulturen.

Die Verbindungen der Formel I sind im allgemeinen wasserunlöslich und können nach irgendeiner der für unlösliche Verbindungen üblichen Methoden konfektioniert werden.

Wenn gewünscht, können die Verbindungen der Formel I in einem mit Wasser nicht mischbaren Lösungsmittel, wie beispielsweise einem hochsiedenden Kohlenwasserstoff, gelöst werden, das zweckmässigerweise gelöste Emulgatoren enthält, so das es bei Zugabe zu Wasser als selbstemulgierbares Oel wirkt.

Die Verbindungen der Formel I können auch mit einem Netzmittel mit oder ohne inertes Verdünnungsmittel zur Bildung eines netzbaren Pulvers vermischt werden, welches in Wasser löslich oder dispergierbar ist, oder sie können mit dem inerten Verdünnungsmittel zur Bildung eines festen oder pulverförmigen Produktes vermischt werden.

Inerte Verdünnungsmittel, mit welchen die Verbindungen der Formel I verarbeitet werden können, sind feste inerte Medien, einschliesslich pulverförmige oder feinverteilte Feststoffe, wie beispielsweise Tone, Sande, Talk, Glimmer, Düngemittel und dgl., wobei solche Produkte entweder staubförmig oder als Materialien mit grösserer Teilchengrösse vorliegen können.

Die Netzmittel können anionische Verbindungen, wie beispielsweise Seifen, Fettsulfatester, die Dodecylnatriumsulfat, Octadecylnatriumsulfat und Cetylnatriumsulfat, fettaromatische Sulfonate, wie Alkylbenzolsulfonate oder Butylnaphthalinsulfonate, komplexere Fettsulfonate, wie die Amidkondensationsprodukte von Oelsäure und N-Methyltaurin oder das Natriumsulfonat von Dioctylsuccinat sein.

Die Netzmittel können auch nichtionische Netzmittel, wie beispielsweise Kondensationsprodukte von Fettsäuren, Fettalkoholen oder fettsubstituierten Phenolen mit Aethylenoxyd, oder Fettsäureester und -äther von Zuckern oder mehrwertigen Alkoholen oder die Produkte, die aus letzteren durch Kondensation mit Aethylenoxyd erhalten werden, oder die Produkte, die als Blockcopolymere von Aethylenoxyd und Propylenoxyd bekannt sind, sein. Die Netzmittel können auch kationische Mittel, wie beispielsweise Cetyltrimethylammoniumbromid und dgl., sein.

Die herbicide Zusammensetzung kann auch in Form einer Aerosolverbindung vorliegen, wobei zweckmässigerweise zusätzlich zum Treibgas, welches geeigneterweise ein polyhalogeniertes Alkan, wie Dichlordifluormethan ist, ein Co-Solvens und ein Netzmittel verwendet wird.

Die herbiciden Zusammensetzungen, gemäss der vorliegenden Erfindung können zusätzlich zu den Verbindungen der Formel I, Synergisten und andere aktive Insektizide, Bakterizide, Herbicide und Fungizide enthalten.

In ihren verschiedenen Anwendungsgebieten können die erfindungsgemässen Verbindungen in unterschiedlichen Mengenverhältnissen eingesetzt werden.

Die erfindungsgemässen Unkrautbekämpfungsmittel können in einer Form vorliegen, die sich für die Lagerung und den Transport eignen. Solche Formen können z.B. 2—90% an einem oder mehreren Wirkstoffen der Formel I enthalten. Diese Formen können dann mit gleichen oder verschiedenen Trägermaterialien bis zu Konzentrationen verdünnt werden, die sich für den praktischen Gebrauch eignen. Im gebrauchsfertigen Mittel können dann Wirkstoffkonzentrationen von 2—80% (Gewichtsprozent) vorliegen. Die Wirkstoffkonzentration kann jedoch auch kleiner oder grösser sein. Je nach Verwendungszweck ist eine Wirkstoffkonzentration von 2—8% bzw. 50—80% besonders bevorzugt.

Ausgangsmaterialien zur Herstellung ganz besonders bevorzugter Verbindungen der Formel I sind optisch aktive Verbindungen der D-Reihe der allgemeinen Formel

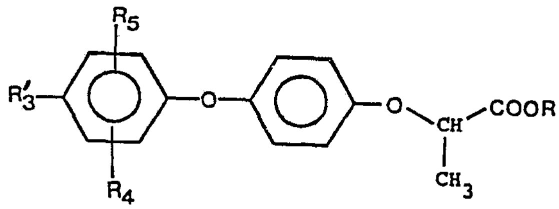

worin R Wasserstoff oder Alkyl mit 1—6 Kohlenstoffatomen, $R'_3$ Wasserstoff, Fluor, Brom, Trifluormethyl oder Nitro und $R_4$ und $R_5$ die in Formel I angegebene Bedeutung besitzen, mit der Massgabe, dass R Alkyl mit 1—6 Kohlenstoffatomen bedeutet, wenn $R_3'$ Trifluormethyl ist.

Besonders bevorzugte Ausgangsmaterialien sind:

D-2-[p-(p-Bromphenoxy)phenoxy]-propionsäure,
D-2-[p-(p-Fluorphenoxy)phenoxy]-propionsäure,
D-2-[D-(p-Bromphenoxy)phenoxy]-propionsäureäthylester,
D-2-[p-(p-Nitrophenoxy)phenoxy]-propionsäure,
D-2-[p-(p-Trifluormethylphenoxy)phenoxy]-propionsäureäthylester,
D-2-[p-(p-Trifluormethylphenoxy)phenoxy]-propionsäuremethylester.

Die folgenden Beispiele sollen die vorliegende Erfindung illustrieren. Alle Temperaturen sind in °C angegeben.

Beispiel 1

1,1 g einer 50%igen Suspension von Natriumhydrid in Mineralöl werden in einer Inertgasatmosphäre zweimal mit je 5,0 ml Tetrahydrofuran gewaschen, dann in 15,0 ml Tetrahydrofuran eingetragen und tropfenweise mit einer Lösung von 5,0 g p-(p-Chlorphenoxy)phenol, gelöst in 30,0 ml Dimethylformamid, versetzt. In das Gemisch werden anschliessend 5,2 g 2-Brompropionylacetonoxim in 20,0 ml Dimethylformamid eingetropft. Das Reaktionsgemisch wird 2 Stunden unter Rückflussbedingungen erhitzt, danach abgekühlt, auf Eis gegossen und erschöpfend mit Aether extrahiert. Der Aetherextrakt wird mit Wasser gewaschen, über Natriumsulfat getrocknet und unter vermindertem Druck eingedampft. Das zurückbleibende Aceton-O-/2-[p-(p-chlorphenoxy)phenoxy]propionyl/-oxim kann durch Adsorption an Kieselgel gereinigt werden: $n_D^{20}$: 1,5583.

In analoger Weise erhält man beim Einsatz von:

p-(p-Chlorphenoxy)phenol und 2-Brom-propionylacetophenonoxim das Acetophenon O-/2-[p-(p-Chlorphenoxy)phenoxy]propionyl/-oxim; $n_D^{20}$: 1,5921.

p-(p-Chlorphenoxy)phenol und 2-Brom-propionyl-2-butanonoxim das 2-Butanon O-/2-[p-(p-Chlorphenoxy)phenoxy]propinyl/-oxim; $n_D^{20}$: 1,5510.

p-(p-Trifluormethylphenoxy)phenol und 2-Brom-propionyl-2-butanonoxim das 2-Butanon O-/2-[p-(p-Trifluormethylphenoxy)phenoxy]propionyl/-oxim; $n_D^{20}$: 1,5169.

p-(p-Trifluormethylphenoxy)phenol und 2-Brom-propionylacetonoxim das Aceton-O-/2-[p-(p-trifluormethylphenoxy)phenoxy]propionyl/-oxim; $n_D^{20}$: 1,5212.

p-(p-Trifluormethylphenoxy)phenol und 2-Brom-propionylacetophenonoxim das Acetophenon-O-/2-[p-(p-trifluormethylphenoxy)phenoxy]propionyl/-oxim; Fp.: 102—103°.

p-(p-Trifluormethylphenoxy)phenol und 2-Brompropionyl-3,5,5-trimethyl-2-cyclohexenonoxim das 3,5,5-Trimethyl-2-cyclohexanon-O-/2-[p-(p-trifluormethylphenoxy)phenoxy]propionyl/-oxim; $n_D^{20}$: 1,5335.

p-(p-Trifluormethylphenoxy)phenol und 2-Brompropionylcyclohexanonoxim das Cyclohexanon-O-/2-[p-(p-trifluormethyl-phenoxy)phenoxy]propionyl/-oxim; $n_D^{20}$: 1,5251.

p-(p-Trifluormethylphenoxy)phenol und 2-Brompropionyl-6-undecanonoxim das 6-Undecanon-O-/2-[p-(p-trifluormethylphenoxy)phenoxy]propionyl/-oxim; $n_D^{20}$: 1,4999.

Das Ausgangsmaterial kann wie folgt hergestellt werden:

5,0 g Acetonoxim und 5,6 g Pyridin werden in 50 ml Tetrahydrofuran gelöst. Bei 0°C werden nun 15,0 g 2-Brom-propionsäurebromid zugetropft. Danach wird 3 Stunden bei Raumtemperatur nachgerührt, auf Eis gegossen, und mit Aether extrahiert. Der Aetherextrakt wird mit Wasser gewaschen, über Natriumsulfat getrocknet und unter vermindertem Druck eingedampft. Das zurückbleibende 2-Brompropionylacetonoxim kann durch Adsorption an Kieselgel gereinigt werden. $n_D^{20}$: 1,4829.

In analoger Weise erhält man beim Einsatz von:

2-Butanoxim und 2-Brompropionylbromid das 2-Brompropionyl-2-butanonoxim; $n_D^{20}$: 1.4840.

Acetophenon und 2-Brompropionylbromid das 2-Brompropionyl-acetophenonoxim; $n_D^{20}$: 1,5650.

3,5,5-Trimethyl-2-cyclohexenonoxim und 2-Brompropionylbromid das 2-Brompropionyl-3,5,5-trimethyl-2-cyclohexenonoxim; $n_D^{20}$: 1,5291.

Cyclohexanonoxim und 2-Brompropionylbromid das 2-Brompropionylcyclohexanonoxim; $n_D^{20}$: 1,5146.

6-Undecanon und 2-Brompropionylbromid das 2-Brompropionyl-6-undecanonoxim; $n_D^{20}$: 1,4760.

Das 3-Butin-2-on-oxim kann wie folgt hergestellt werden:

2,5 g 3-Butin-2-on, gelöst in 10 ml Aethanol, werden bei 20° zu einer Lösung von 5,1 g Hydroxylamin-hydrochlorid und 1,6 g Natriumhydroxid in 30 ml Wasser zugetropft. Danach wird 2 Stunden bei Raumtemperatur nachgerührt, auf 150 ml Wasser gegossen und mit Dichlormethan extrahiert. Der Extrakt wird mit Wasser gewaschen, über Natriumsulfat getrocknet und eingedampft. Das zurückbleibende 3-Butin-2-onoxim kann durch Adsorption an Kieselgel gereinigt werden; $n_D^{20}$: 1,424.

Beispiel 2

20 g D-2-[p-(p-Chlorphenoxy)phenoxy]propionsäure werden mit 8,7 g Oxalylchlorid und 100 ml Benzol 3 Stunden bei 40°C gerührt. Dann destilliert man Benzol im Vakuum ab. Zum erhaltenen Säurechlorid wurden 5 g Acetonoxim, 5,0 g Pyridin und 50 ml Tetrahydrofuran gegeben und das Gemisch 2 Stunden unter Rühren bei Raumtemperatur umgesetzt. Das Reaktionsprodukt wird in Wasser gegossen und mit Aether extrahiert. Der Aetherextrakt wird mit Wasser gewaschen, über Natriumsulfat getrocknet und unter vermindertem Druck eingedampft. Das zurückbleibende Aceton-O-D-/2-[p-(p-chlorphenoxy)phenoxy]propionyl/-oxim kann durch Adsorption an Kieselgel gereinigt werden. $n_D^{20}$: 1,5583.

In analoger Weise erhält man beim Einsatz von:

D-2-[p-(p-Trifluormethylphenoxy)phenoxy]propionsäure und Acetonoxim das Aceton-O-D-/2-[p-(p-trifluormethylphenoxy)phenoxy]propionyl/-oxim; die Struktur wurde durch Kernresonanzspektrum bestätigt, $[\alpha]_D^{20}$ +50,8° ($CHCl_3$, 1%).

2-[p-(p-Trifluormethylphenoxy)phenoxy]propionsäure und 2,6-Dichlorbenzaldehydoxim das 2,6-Dichlorbenzaldehyd-O-/2-[p-(p-trifluormethylphenoxy)phenoxy]propionyl/-oxim. $n_D^{20}$: 1,5679.

2-[p-(p-Trifluormethylphenoxy)phenoxy]propionsäure und Benzaldehydoxim das Benzaldehyd-O-/2-[p-(p-trifluormethylphenoxy)phenoxy]propionyl/-oxim, Smp. 102—103°.

D-2-[p-(p-Trifluormethylphenoxy)phenoxy]propionsäure und Acetophenonoxim das Acetophenon-O-D-/2-[p-(p-trifluormethylphenoxy)phenoxy]propionyl/-oxim, Smp. 56—57°.

2-[p-(p-Chlorphenoxy)phenoxy]propionsäure und Benzaldehydoxim das Benzaldehyd-O-2-[p-(p-Chlorphenoxy)phenoxy]propionyl-oxim, Smp. 116—117°.

2-[p-(p-Trifluormethylphenoxy)phenoxy]propionsäure und Heptan-4-onoxim das Heptan-4-on-O-2-[p-(p-trifluormethylphenoxy)phenoxy]propionyloxim. $n_D^{20}$: 1,5101.

2-[p-(p-Trifluormethylphenoxy)phenoxy]propionsäure und Isopropylmethylketonoxim das 2-Methylbutan-3-on-O-2-[p-(p-trifluormethylphenoxy)phenoxy]propionyloxim. $n_D^{20}$: 1,5105.

2-[p-(p-Trifluormethylphenoxy)phenoxy]propionsäure und 4-Methyl-3-penten-2-on-oxim das 4-Methyl-3-penten-2-on-O-2-[p-(p-trifluormethylphenoxy)phenoxy]propionyloxim. $n_D^{20}$: 1,5295.

2-[p-(p-Chlorphenoxy)phenoxy]propionsäure und 6-Methyl-5-hepten-2-onoxim das 6-Methyl-5-hepten-2-on-O-2-[p-(p-chlorphenoxy)phenoxy]propionyloxim. $n_D^{20}$: 1,5527.

2-[p-(p-Chlorphenoxy)phenoxy]propionsäure und Cyclohexanonoxim das Cyclohexanon-O-2-[p-(p-chlorphenoxy)phenoxy]propionyloxim. $n_D^{20}$: 1,5658.

2-[p-(p-Bromphenoxy)phenoxy]propionsäure und Acetonoxim das Aceton-O-2-[p-(p-bromphenoxy)phenoxy]propionyloxim. $n_D^{20}$: 1,5610.

2-[p-(p-Trifluormethylphenoxy)phenoxy]propionsäure und 3-Butin-2-onoxim das 3-Butin-2-on-O-/2-[p-(p-trifluormethylphenoxy)phenoxy]propionyloxim. $n_D^{20}$: 1,5480.

## Beispiel 3

4 g 2-[p-(p-Trifluormethylphenoxy)phenoxy]propionylchlorid werden zu einer Lösung von 0,8 g Acetonoxim und 1 ml Pyridin in 20 ml Tetrahydrofuran zugetropft. Danach wird 3 Stunden bei Raumtemperatur nachgerührt, auf Eis gegossen und mit Aether extrahiert. Der Aetherextrakt wird mit Wasser gewaschen, über Natriumsulfat getrocknet und unter vermindertem Druck eingedampft. Das zurückbleibende Aceton-O-/2-[p-(p-trifluormethylphenoxy)phenoxy]propionyl/-oxim kann durch Adsorption an Kieselgel gereinigt werden. $n_D^{20}$: 1,5212.

## Beispiel 4

15 g 2-[p-(p-Trifluormethylphenoxy)phenoxy]propionsäure werden in 20 ml Dichlormethan suspendiert und bei Raumtemperatur werden 3,5 g Acetonoxim zugegeben. Danach werden 9,8 g Dicyclohexylcarbodiimid, gelöst in 10 ml Dichlormethan, über einen Zeitraum von 10 Minuten, zugetropft, dabei steigt die Temperatur bis gegen 40°C an. Es wird 2 Stunden bei Raumtemperatur nachgerührt. Das Reaktionsgemisch wird filtriert und am Rotationsverdampfer zur Trockene eingedampft. Man erhält so Aceton-O-/2-[p-(p-trifluormethylphenoxy)phenoxy]propionyl/-oxim, $n_D^{20}$: 1,5212.

## Beispiel 5

10 g 2-[p-(p-Trifluormethylphenoxy)phenoxy]propionsäure werden mit 10 g Thionylchlorid und 2 Tropfen Dimethylformamid 2 Stunden am Rückfluss erhitzt. Dann destilliert man das überschüssige Thionylchlorid am Wasserstrahl-Vakuum ab. Danach erhält man 2-[p-(p-Trifluormethylphenoxy)-phenoxy]propionsäurechlorid.

10,5 g des so hergestellten Säurechlorids werden in 10 ml Toluol gelöst und mit einer Lösung von 2,4 g Acetonoxim in 3,03 g Triäthylamin und 20 ml Toluol versetzt. Es wird 1 Stunde nachgerührt und mit zweimal 50 ml, insgesamt 100 ml Wasser nachgewaschen, über Natrium sulfat getrocknet und eingeengt. Man erhält so das Aceton-O-/2-[p-(p-trifluormethylphenoxy)phenoxy]propionyl/-oxim, $n_D^{20}$: 1,5260.

## Beispiel 6

Herstellung von Ausgangsmaterialien der Formel II:

Nachstehend werden zwei beispielhafte Möglichkeiten für die Herstellung der optisch aktiven Ausgangsmaterialien angegeben:

1. Herstellung des D-2-[p-Trifluormethylphenoxy)phenoxy]propionsäureäthylesters und D-2-[p-(p-Chlorphenoxy)phenoxy]propionsäureäthylesters.

50 g p-(p-Trifluormethylphenoxy)phenol werden mit 38,4 g L(-)-2-(Methylsulfonyloxy)propionsäureäthylester vermischt und danach werden 11,4 g wasserfreies Soda zugegeben. Das Gemisch wird 18 Stunden bei 110°C gerührt, dann abgekühlt und mit 300 ml Aether versetzt. Die organische Phase wird mit 2 N NaOH und Wasser ausgewaschen, über Natriumsulfat getrocknet und eingeengt. Man erhält D-2-[p-(p-Trifluormethylphenoxy)phenoxy]propionsäureäthylester; $[\alpha]_D^{22} = +19{,}2°$ ($CHCl_3$; c = 1,05%).

In analoger Weise erhält man bei Einsatz von:

p-(p-Chlorphenoxy)phenol und L(-)-2-(Methylsulfonyloxy)propionsäureäthylester den D-2-[p-(p-Chlorphenoxy)phenoxy]propionsäureäthylester; $[\alpha]_D^{20} = +28{,}7°$ ($CHCl_3$; c = 1,05%).

2. Herstellung der entsprechenden Säuren durch Esterverseifung.

58 g D-2-[p-(p-Trifluormethylphenoxy)phenoxy]propionsäureäthylester werden in 50 ml Methanol suspendiert, mit 27 g KOH (gelöst in 50 ml Wasser) versetzt und während 1 1/2 Stunden bei Raumtemperatur gerührt. Zur Aufarbeitung wird das Gemisch mit 200 ml Wasser verdünnt und mit HCl angesäuert. Die ausgefallene D-2-[p-(p-Trifluormethylphenoxy)phenoxy]propionsäure wird abfiltriert, mit Wasser gewaschen und getrocknet. $[\alpha]_D^{22} = +12,88°$ ($CHCl_3$; c = 1,1%).

In analoger Weise erhält man beim Einsatz von:

D-2-[p-(p-Chlorphenoxy)phenoxy]propionsäureäthylester die D-2-[p-(p-Chlorphenoxy)phenoxy]-propionsäure, $[\alpha]_D^{20} = +20.1°$ ($CHCl_3$; c = 1,01%).

Beispiel 7

Zur Herstellung eines emulgierbaren Konzentrates werden die nachstehend aufgeführten Bestandteile dieses Konzentrates miteinander vermischt:

| | |
|---|---|
| Verbindung der Formel I | 500 g |
| Kondensationsprodukte eines Alkylphenoles und Aethylenoxyd; Dodecylbenzolsulfonsaures Calcium | 100 g |
| Epoxydiertes Soyaöl mit einem Oxiransauerstoffgehalt von ca. 6% | 25 g |
| Butyliertes Hydroxytoluol | 10 g |

Diese Mischung wird mit Xylol auf 1 Liter aufgefüllt.

Beispiel 8

Der Wirkstoff, beispielsweise Aceton-O-/2-[p-(p-chlorphenoxy)phenoxy]propionyl/-oxim (Verbindung A in der nachstehenden Tabelle), wird so in Aceton gelöst, dass eine 2% Lösung an Wirkstoff entsteht. Kurz vor Versuchsbeginn wird mit Wasser auf die gewünschte Konzentration verdünnt, die im vorliegenden Versuch 156—1250 g/ha beträgt. (Bei Verwendung von unlöslichen Wirkstoffen werden Spritzpulver formuliert, die Kaolin als inertes Verdünnungsmittel enthalten).

Die Testpflanzen werden im Gewächshaus mit dem formulierten Wirkstoff besprüht; mittels Quecksilberdampflampen wird ein 16 Stunden Tag simuliert. 3 Wochen nach dem Besprühen werden die Pflanzen auf die eingetretene Wirkung untersucht, d.h. es werden die % Nekrose bestimmt, wobei 100% Nekrose einer vollständigen Zerstörung der Pflanze entspricht. Lösungsmitteleffekte — soweit vorhanden — werden mittels der "Abbott-Formel" kompensiert. Die Resultate sind in der nachstehenden Tabelle zusammengefasst.

TABELLE I (% Nekrose)

| Wirkstoff | Dosierung g/ha | Alopecurus myosuroides | Digitaria floridana | Avena fatua |
|---|---|---|---|---|
| Verbindung A | 625 | 80 | 60 | — |
| B | 625 | 100 | 40 | — |
| C | 625 | 100 | 100 | 100 |
| | 156 | 50 | 100 | 20 |
| D | 625 | 100 | 100 | 100 |
| | 156 | 100 | 100 | 100 |
| E | 625 | 100 | 100 | 70 |
| F | 625 | 70 | 100 | 50 |
| G | 625 | 100 | 100 | 70 |

A = Aceton-O-/2-[p-(p-chlorphenoxy)phenoxy]propionyl/-oxim
B = Aceton-O-/2-[p-(p-bromphenoxy)phenoxy]propionyl/-oxim
C = Aceton-O-/2-[p-(p-trifluormethylphenoxy)phenoxy]propionyl/-oxim
D = Aceton-O-/D-2-[p-(p-trifluormethylphenoxy)phenoxy]propionyl/-oxim
E = Acetophenon-O-/D-2-[p-(p-trifluormethylphenoxy)phenoxy]propionyl/-oxim
F = Cyclohexanon-O-/2-[p-(p-trifluormethylphenoxy)phenoxy]propionyl/-oxim
G = 2-Butanon-O-/2-[p-(p-trifluormethylphenoxy)phenoxy]propionyl/-oxim

Beispiel 9

TABELLE II

% Necrosis bei Bromus secalinus

| Dosierung kg/ha | D | C |
|---|---|---|
| 0,15 | 80 | 0 |
| 0,31 | 90 | 0 |

| | | |
|---|---|---|
| *Formulierung:* | Wirkstoff | 250 g/l |
| | NMP (N-Methyl-2-pyrrolidon) | 300 g/l |
| | Tensiofix B 7425® | 100 g/l |
| | Phenylsulfonat CA® | 25 g/l |
| | Shellsol AB® auf auffüllen. | 1000 ml |
| *Netzmittel:* | 0,08% Nonoxynol (Kondensations produkt aus Nonylphenol und Aethylenoxyd) | |
| *Wachstumsstadium bei Behandlung:* | 6—9 Blattstadium | |
| *Sprühvolumen:* | 1000 l/ha | |
| *Bonitierung:* | 3 Wochen nach Behandlung | |
| *Ort:* | Gewächshaus | |

Verbindung C siehe Tabelle I
Verbindung D siehe Tabelle I

Beispiel 10

TABELLE III

% Necrosis bei Baumwolle cv. Stoneville 7A

| Dosierung kg/ha | D | C |
|---|---|---|
| 1,25 | 0 | 18 |
| 2,5 | 28 | 50 |

| | | | |
|---|---|---|---|
| *Formulierung:* | Wirkstoff | | 250 g/l |
| | NMP | | 300 g/l |
| | Tensiofix B 7425® | | 100 g/l |
| | Phenylsulfonat CA® | | 25 g/l |
| | Shellsol AB® auffüllen. | auf | 1000 ml |
| *Netzmittel:* | 0.08% Nonoxynol | | |
| *Wachstumsstadium bei Behandlung:* | 2 Blattstadium | | |
| *Sprühvolumen:* | 1000 l/ha | | |
| *Bonitierung:* | 10 Tage nach behandlung | | |
| *Ort:* | Gewächshaus | | |

Verbindung C siehe Tabelle I
Verbindung D siehe Tabelle I

Beispiel 11

TABELLE IV

% Necrosis bei Soyabohnen cv. Hood

| Dosierung kg/ha | D | C |
|---|---|---|
| 2,25 | 0 | 10 |
| 2,5 | 0 | 25 |

| | | | |
|---|---|---|---|
| *Formulierung:* | Wirkstoff | | 250 g/l |
| | NMP | | 300 g/l |
| | Tensiofix B 7425® | | 100 g/l |
| | Phenylsulfonat CA® | | 25 g/l |
| | Shellsol AB® auffüllen. | auf | 1000 ml |
| *Netzmittel:* | 0.08% Nonoxynol | | |
| *Wachstumsstadium bei Behandlung:* | 2 dreiblättrige Blätter | | |
| *Sprühvolumen:* | 1000 l/ha | | |
| *Bonitierung:* | 2 Wochen nach Behandlung | | |
| *Ort:* | Gewächshaus | | |

Verbindung C siehe Tabelle I
Verbindung D siehe Tabelle I

**Patentansprüche**

1. Verbindungen der allgemeinen Formel

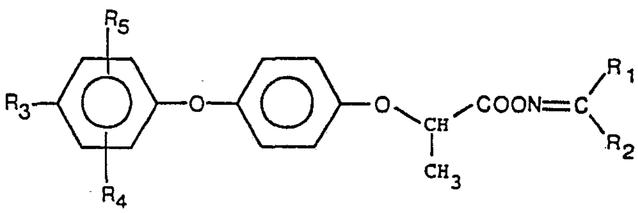

I

worin $R_1$ Wasserstoff, Alkyl mit 1—6 Kohlenstoffatomen oder Phenyl, $R_2$ Wasserstoff, Alkyl mit 1—6 Kohlenstoffatomen, Alkenyl mit 2—6 Kohlenstoffatomen, Alkinyl mit 2—6 Kohlenstoffatomen oder Phenyl oder $R_1$ und $R_2$ zusammen mit dem Kohlenstoffatom, an das sie geknüpft sind, einen Cyclohexanring bilden, der gegebenenfalls mono-, di- oder tri-Alkyl mit 1—3 Kohlenstoffatomen substituiert sein kann, $R_3$ Wasserstoff, Fluor, Chlor, Brom, Trifluormethyl oder Nitro und $R_4$ und $R_5$ Wasserstoff oder Chlor darstellen, mit der Massgabe, das $R_1$ und $R_2$ nicht gleichzeitig Wasserstoff bedeuten.

2. Verbindungen der Formel I in Anspruch 1, worin $R_1$ und $R_2$ Alkylreste mit 1—2 Kohlenstoffatomen oder einen Phenylrest und $R_3$ Chlor oder den Trifluormethylrest und $R_4$ und $R_5$ Wasserstoff bedeuten.

3. Verbindungen der allgemeinen Formel I in Anspruch 1, worin $R_1$ und $R_2$ eine Methylgruppe und $R_3$ Chlor oder den Trifluormethylrest und $R_4$ und $R_5$ Wasserstoff bedeuten.

4. Aceton-O-/2-[p-(p-chlorphenoxy)phenoxy]propionyl/-oxim.

5. Aceton-O-D-/2-[p-(p-chlorphenoxy)phenoxy]propionyl/-oxim.

6. Aceton-O-/2-[p-(p-trifluormethylphenoxy)phenoxy]propionyl/-oxim.

7. Aceton-O-D-/2-[p-(p-trifluormethylphenoxy)phenoxy]propionyl/-oxim.

8. Aceton-O-/2-[p-(p-bromphenoxy)phenoxy]propionyl/-oxim.

9. Optisch aktive Verbindungen der D-Reihe entsprechend den Verbindungen in den Ansprüchen 1—4, 6 und 8.

10. Eine Verbindung gemäss einem der Ansprüche 1—9 als Unkrautbekämpfungsmittel.

11. Unkrautbekämpfungsmittel, dadurch gekennzeichnet, dass es mindestens eine Verbindung der allgemeinen Formel I in Anspruch 1 und inertes Trägermaterial enthält.

12. Unkrautbekämpfungsmittel nach Anspruch 11, dadurch gekennzeichnet, dass es Aceton-O-/2-[p-(p-chlorphenoxy)phenoxy]propionyl/-oxim enthält.

13. Unkrautbekämpfungsmittel nach Anspruch 11, dadurch gekennzeichnet, dass es Aceton-O-D-/2-[p-(p-chlorphenoxy)phenoxy]propionyl/-oxim enthält.

14. Unkrautbekämpfungsmittel nach Anspruch 11, dadurch gekennzeichnet, dass es Aceton-O-/2-[p-(p-trifluormethylphenoxy)phenoxy]propionyl/-oxim enthält.

15. Unkrautbekämpfungsmittel nach Anspruch 11, dadurch gekennzeichnet, dass es Aceton-O-D-/2-[p-(p-trifluormethylphenoxy)phenoxy]propionyl/-oxim enthält.

16. Verfahren zur Herstellung von Verbindungen der allgemeinen Formel I in Anspruch 1, dadurch, gekennzeichnet, dass man

a) ein Säurechlorid oder ein reaktionsfähiges Anhydrid dieser Säure der Formel

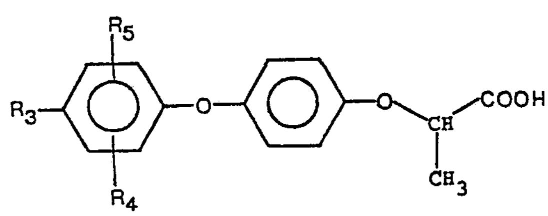

II

worin $R_3$, $R_4$ und $R_5$ die in Anspruch 1 angegebene Bedeutung besitzen, mit einem Oxim

$$R_1R_2CNOH \qquad III$$

worin $R_1$ und $R_2$ die in Anspruch 1 angegebene Bedeutung besitzen,

umsetzt, oder

b) eine Verbindung der allgemeinen Formel

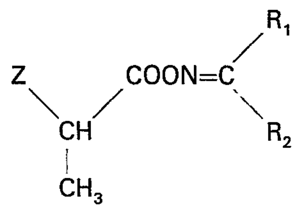

IV

worin Z Chlor, Brom, Jod, Mesyloxy oder Tosyloxy bedeutet und $R_1$ und $R_2$ die in Anspruch 1 angegebene Bedeutung besitzen,

mit einer Verbindung der allgemeinen Formel

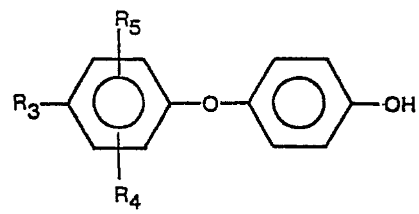

V

worin $R_3$, $R_4$ und $R_5$ die in Anspruch 1 angegebene Bedeutung besitzen,

oder einem Alkalimetallsalz davon, notwendigenfalls in Gegenwart einer Base, umsetzt, oder

c) eine Säure der Formel II mit einem Oxim der Formel III in Gegenwart von Dicyclohexylcarbodiimid umsetzt.

17. Verfahren nach Anspruch 16 zur Herstellung von Aceton-O-/2-[p-(p-trifluormethylphenoxy)-phenoxy]propionyl/-oxim, dadurch gekennzeichnet, dass man 2-[p-(p-Trifluormethylphenoxy)-phenoxy]-propionsäure mit Acetonoxim in Gegenwart von Dicyclohexylcarbodiimid umsetzt.

18. Verfahren nach Anspruch 16 zur Herstellung von Aceton-O-/2-[p-(p-trifluormethylphenoxy)-phenoxy]-propionyl/-oxim, dadurch gekennzeichnet, dass man p-(p-Trifluormethylphenoxy)phenol und 2-Brom-propionylacetonoxim umsetzt.

19. Verfahren nach Anspruch 16 zur Herstellung von Aceton-O-D-/2-[p-(p-trifluormethyl-phenoxy)phenoxy]propionyl/-oxim, dadurch gekennzeichnet, dass man D-2-[p-(p-Trifluor-methylphenoxy]-propionsäurechlorid mit Acetonoxim umsetzt.

20. Verwendung der in den Ansprüchen 11—15 genannten Verbindungen als Unkrautbekämpfungsmittel.

**Revendications**

1. Composés de formule générale

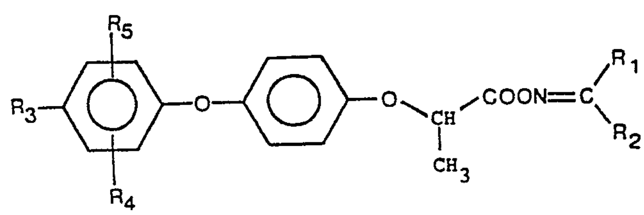

I

dans laquelle $R_1$ représente l'hydrogène, un groupe alkyle contenant de 1 à 6 atomes de carbone ou un groupe phényle, $R_2$ représente l'hydrogène, un groupe alkyle contenant de 1 à 6 atomes de carbone, alcényle contenant de 2 à 6 atomes de carbone, alcynyle contenant de 2 à 6 atomes de carbone ou phényle ou bien $R_1$ et $R_2$ forment ensemble et avec l'atome de carbone auquel ils sont fixés un noyau cyclohexane qui peut éventuellement porter un, deux ou trois substituants alkyles en C1—C3, $R_3$ représente l'hydrogène, le fluor, le chlore, le brome, un groupe trifluorométhyle ou nitro et $R_4$ et $R_5$ représentent l'hydrogène ou le chlore, étant spécifié que $R_1$ et $R_2$ ne peuvent représenter en même temps l'hydrogène.

2. Composés de formule I selon la revendication 1 dans lesquels $R_1$ et $R_2$ représentent des groupes alkyles en C1—C2 ou un groupe phényle et $R_3$ représente le chlore ou le groupe trifluorométhyle et $R_4$ et $R_5$ représentent l'hydrogène.

3. Composés de formule générale I selon la revendication 1 dans lesquels $R_1$ et $R_2$ représentent un groupe méthyle et $R_3$ représente le chlore ou le groupe trifluorométhyle et $R_4$ et $R_5$ représentent l'hydrogène.

4. L'acétone-O-/2-[p-(p-chlorophénoxy)-phénoxy]propionyl/-oxime.

5. L'acétone-O-D-/2-[p-(p-chlorophénoxy)-phénoxy]propionyl/-oxime.

6. L'acétone-O-/2-[p-(p-trifluorométhylphénoxy)-phénoxy]-propionyl/-oxime.

7. L'acétone-O-D-/2-[p-(p-trifluorométhylphénoxy)-phénoxy]-propionyl/-oxime.

8. L'acétone-O-/2-[p-(p-bromophénoxy)phénoxy]propionyl/-oxime.

9. Composés énantiomères de la série D correspondant aux composés des revendications 1 à 4, 6 et 8.

10. Un composé selon l'une des revendications 1 à 9 en tant que produit herbicide.

11. Produit herbicide caractérisé en ce qu'il contient au moins un composé de formule générale I de la revendication 1 et une matière de support inerte.

12. Produit herbicide selon la revendication 11, caractérisé en ce qu'il contient de l'acétone-O-/2-[p-(p-chlorophénoxy)-phénoxy]propionyl/-oxime.

13. Produit herbicide selon la revendication 11, caractérisé en ce qu'il contient de l'acétone-O-D-/2-[p-(p-chlorophénoxy)-phénoxy]propionyl/-oxime.

14. Produit herbicide selon la revendication 11, caractérisé en ce qu'il contient de l'acétone-O-/2-[p-(p-trifluorométhylphénoxy)-phénoxy]propionyl/-oxime.

15. Produit herbicide selon la revendication 11, caractérisé en ce qu'il contient de l'acétone-O-D-/2-[p-(p-trifluorométhylphénoxy)phénoxy]propionyl/-oxime.

16. Procédé de préparation des composés de formule générale I de la revendication 1, caractérisé en ce que

a) on fait réagir un chlorure d'acide ou un anhydride réactif de cet acide de formule

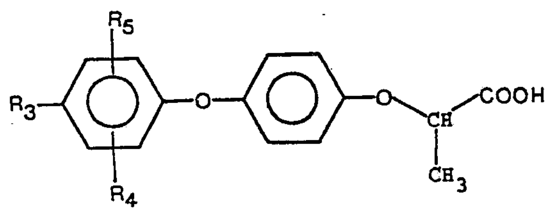

II

dans laquelle $R_3$, $R_4$ et $R_5$ ont les significations indiquées dans la revendication 1, avec une oxime

$$R_1R_2CNOH \qquad III$$

dans laquelle $R_1$ et $R_2$ ont les significations indiquées dans la revendication 1, ou bien

b) on fait réagir un composé de formule générale

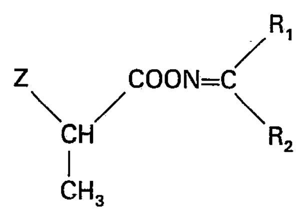

IV

dans laquelle Z représente le chlore, le brome, l'iode, un groupe mésyloxy ou tosyloxy et $R_1$ et $R_2$ ont les significations indiquées dans la revendication 1,
avec un composé de formule générale

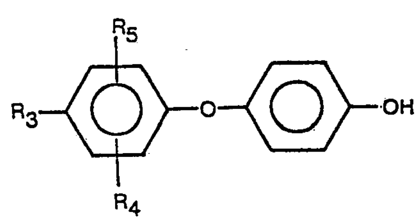

V

dans laquelle $R_3$, $R_4$ et $R_5$ ont les significations indiquées dans la revendication 1,
ou un sel de métal alcalin de ce composé, en présence d'une base lorsque c'est nécessaire, ou bien

c) on fait réagir un acide de formule II avec un oxime de formule III en présence du dicyclohexylcarbodiimide.

17. Procédé selon la revendication 16 pour préparer l'acétone-O-/2-[p-(p-trifluorométhylphénoxy)phénoxy]propionyl/-oxime, caractérisé en ce que l'on fait réagir l'acide 2-[p-(p-trifluorométhylphénoxy)phénoxy]propionique avec l'acétonoxime en présence du dicyclohexylcarbodiimide.

18. Procédé selon la revendication 16 pour préparer l'acétone-O-/2-[p-(p-trifluorométhylphénoxy)phénoxy]propionyl/-oxime, caractérisé en ce que l'on fait réagir le p-(p-trifluorométhylphénoxy)-phénol et la 2-bromo-propionylacétonoxime.

19. Procédé selon la revendication 16 pour préparer l'acétone-O-D-/2-[p-(p-trifluorométhylphénoxy)phénoxy]propionyl/-oxime, caractérisé en ce que l'on fait réagir le chlorure de l'acide D-2-[p-(p-trifluorométhylphénoxy)phénoxy]propionique avec l'acétonoxime.

20. Utilisation des composés mentionnés dans les revendications 11 à 15 en tant que produits herbicides.

## Claims

1. Compounds of the general formula

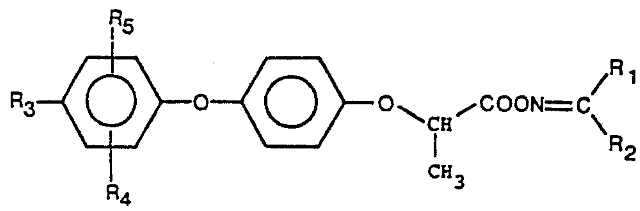

I

wherein $R_1$ represents hydrogen, alkyl with 1—6 carbon atoms or phenyl, $R_2$ represents hydrogen, alkyl with 1—6 carbon atoms, alkenyl with 2—6 carbon atoms, alkynyl with 2—6 carbon atoms or phenyl or $R_1$ and $R_2$ together with the carbon atom to which they are attached form a cyclohexane ring which, if desired, can be mono-, di- or trisubstituted by alkyl with 1—3 carbon atoms, $R_3$ represents hydrogen, fluorine, chlorine, bromine, trifluoromethyl or nitro and $R_4$ and $R_5$ represent hydrogen or chlorine, with the proviso that $R_1$ and $R_2$ do not simultaneously signify hydrogen.

2. Compounds of formula I in claim 1, wherein $R_1$ and $R_2$ signify alkyl residues with 1—2 carbon atoms or a phenyl residue and $R_3$ signifies chlorine or the trifluoromethyl residue and $R_4$ and $R_5$ signify hydrogen.

3. Compounds of general formula I in claim 1, wherein $R_1$ und $R_2$ signify a methyl group and $R_3$ signifies chlorine or the trifluoromethyl residue and $R_4$ and $R_5$ signify hydrogen.

4. Acetone O-/2-[p-(p-chlorophenoxy)-phenoxy]propionyl/oxime.

5. Acetone O-D-/2-[p-(p-chlorophenoxy)phenoxy]propionyl/oxime.

6. Acetone O-/2-[p-(p-trifluoromethylphenoxy)phenoxy]propionyl/oxime.

7. Acetone O-D-/2-[p-(p-trifluoromethylphenoxy)phenoxy]propionyl/oxime.

8. Acetone O-/2-[p-(p-bromophenoxy)phenoxy]propionyl/oxime.

9. Optically active compounds of the D-series corresponding to the compounds in claims 1—4, 6 and 8.

10. A compound according to any one of claims 1—9 as a weed-control agent.

11. Weed-control composition, characterised in that it contains at least one compound of general formula I in claim 1 and inert carrier material.

12. Weed-control composition according to claim 11, characterised in that it contains acetone O-/2-[p-(p-chlorophenoxy)phenoxy]propionyl/oxime.

13. Weed-control composition according to claim 11, characterised in that it contains acetone O-D-/2-[p-(p-chlorophenoxy)phenoxy]propionyl/oxime.

14. Weed-control composition according to claim 11, characterised in that it contains acetone O-/2-[p-(p-trifluoromethylphenoxy)phenoxy]propionyl/oxime.

15. Weed-control composition according to claim 11, characterised in that it contains acetone O-D-/2-[p-(p-trifluoromethylphenoxy)phenoxy]propionyl/oxime.

16. Process for the manufacture of compounds of general formula I in claim 1, characterised by a) reacting an acid chloride or a reactive anhydride of this acid of the formula

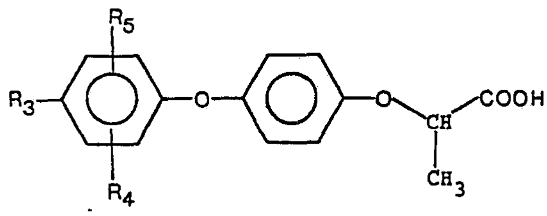

II

wherein $R_3$, $R_4$ and $R_5$ have the significance given in claim 1,
with an oxime

$R_1R_2CNOH$ III

wherein $R_1$ and $R_2$ have the significance given in claim 1,
or

b) reacting a compound of the general formula

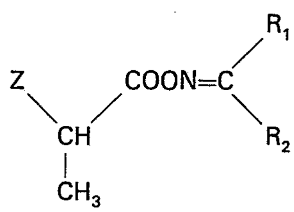

VI

wherein Z signifies chlorine, bromine, iodine, mesyloxy or tosyloxy and $R_1$ and $R_2$ have the significance given in claim 1,
with a compound of the general formula

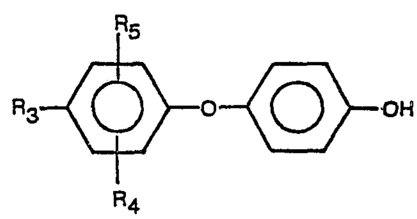

V

wherein $R_3$, $R_4$ and $R_5$ have the significance given in claim 1,
or an alkali metal salt thereof, if necessary in the presence of a base, or

c) reacting an acid of formula II with an oxime of formula III in the presence of dicyclohexylcarbodiimide.

17. Process according to claim 16 for the manufacture of acetone O-/2-[p-(p-trifluoromethylphenoxy)phenoxy]propionyl/oxime, characterised by reacting 2-[p-(p-trifluoromethylphenoxy)-phenoxy]-propionic acid with acetone oxime in the presence of dicyclohexylcarbodiimide.

18. Process according to claim 16 for the manufacture of acetone O-/2-[p-(p-trifluoromethylphenoxy)phenoxy]propionyl/oxime, characterised by reacting p-(p-trifluoromethylphenoxy)phenol and 2-bromo-propionyl-acetone oxime.

19. Process according to claim 16 for the manufacture of acetone O-D-/2-[p-(p-trifluoromethylphenoxy)phenoxy]propionyl/oxime, characterised by reacting D-2-[p-(p-trifluoromethylphenoxy]-propionic acid chloride with acetone oxime.

20. Use of the compounds named in claims 11—15 as weed-control agents.